# EUROPÄISCHE PATENTSCHRIFT

(11) **EP 3 345 034 B1**
(45) Veröffentlichungstag und Bekanntmachung des Hinweises auf die Patenterteilung: **29.01.2020**
(21) Anmeldenummer: 17793562.4
(22) Anmeldetag: 26.09.2017
(51) Int. Cl.: G02B 26/10, A61B 1/00

(54) **ANREGUNG VON FASERN MIT BIEGEPIEZOAKTUATOREN**
FIBRE EXCITATION WITH PIEZO BENDER ACTUATORS
EXCITATION DE FIBRES AU MOYEN D'ACTIONNEURS PIÉZOÉLECTRIQUES À FLEXION

(30) Priorität: 26.09.2016 DE 102016011647
(43) Veröffentlichungstag der Anmeldung: 11.07.2018
(73) Patentinhaber: Blickfeld GmbH, 80339 München (DE)
(72) Erfinder: PETIT, Florian, 81247 München (DE)
(74) Vertreter: Neusser, Sebastian
(86) Internationale Anmeldenummer: PCT/DE2017/100818
(87) Internationale Veröffentlichungsnummer: WO 2018/054429

(56) Entgegenhaltungen:
- DE-A1-102006 046 554
- US-A1- 2010 177 368
- US-A1- 2013 088 764
- US-A1- 2014 303 441
- US-A1- 2014 354 790
- US-A1- 2017 010 461
- SMIDIWICK Q Y J ET AL: "Modeling and control of the resonant fiber scanner of a novel scanning scope", SECOND JOINT EMBS-BMES CONFERENCE 2002. CONFERENCE PROCEEDINGS. 24TH. ANNUAL INTERNATIONAL CONFERENCE OF THE ENGINEERING IN MEDICINE AND BIOLOGY SOCIETY. ANNUAL FALL MEETING OF THE BIOMEDICAL ENGINEERING SOCIETY. HOUSTON, TX, OCT. 23 - 26, 2002; [ANN, Bd. 2, 23. Oktober 2002 (2002-10-23), Seiten 977-978, XP010619915, DOI: 10.1109/IEMBS.2002.1106234 ISBN: 978-0-7803-7612-0
- D. R. RIVERA ET AL: "Compact and flexible raster scanning multiphoton endoscope capable of imaging unstained tissue", PROCEEDINGS OF THE NATIONAL ACADEMY OF SCIENCES, Bd. 108, Nr. 43, 25. Oktober 2011 (2011-10-25), Seiten 17598-17603, XP055208278, ISSN: 0027-8424, DOI: 10.1073/pnas.1114746108
- JEFFREY H. LEACH ET AL: "Monostatic all-fiber scanning LADAR system", APPLIED OPTICS, Bd. 54, Nr. 33, 16. November 2015 (2015-11-16), Seite 9752, XP055423198, WASHINGTON, DC; US ISSN: 0003-6935, DOI: 10.1364/AO.54.009752
- E Piezoelektrische ET AL: "Stack Multilayer-Piezoaktoren P-882 - P-888", , 4. Januar 2018 (2018-01-04), Seite 41, XP055438181, Gefunden im Internet: URL:http://www.dyneos.ch/pdf/Dyneos_Katalo g_Piezoelektrische_Aktoren.pdf [gefunden am 2018-01-04]

## Beschreibung

### TECHNISCHES GEBIET

Die Erfindung betrifft im Allgemeinen die Anregung einer Faser. Die Erfindung betrifft insbesondere die Anregung einer Faser mit einem ersten Biegepiezoaktuator und einem zweiten Biegepiezoaktuator.

### HINTERGRUND

In verschiedenen Gebieten der Technik kann es erstrebenswert sein, eine Faser anzuregen, d.h. eine Bewegung der Faser zu bewirken. Zum Beispiel kann durch die Bewegung der Faser ein Endoskop implementiert werden. Siehe zum Beispiel Rivera, David R., et al. "Compact and flexible raster scanning multiphoton endoscope capable of imaging unstained tissue." Proceedings of the National Academy of Sciences 108.43 (2011): 17598-17603. Die Bewegung der Faser kann auch dazu verwendet werden, gepulstes Laserlicht zu scannen. Siehe zum Beispiel Leach, Jeffrey H., Stephen R. Chinn, and Lew Goldberg. "Monostatic allfiber scanning LADAR system." Applied optics 54.33 (2015): 9752-9757.

Oftmals werden zur Anregung der Faser röhrenförmige Piezoaktuatoren verwendet. Diese können sich beispielsweise entlang einer Längsachse der Faser erstrecken. Siehe z.B. Smithwick, Q. Y. J., et al. "Modeling and control of the resonant fiber scanner of a novel scanning scope." Engineering in Medicine and Biology, 2002. 24th Annual Conference and the Annual Fall Meeting of the Biomedical Engineering Society EMBS/BMES Conference, 2002. Proceedings of the Second Joint. Vol. 2. IEEE, 2002.

In diesem Zusammenhang offenbart die US 2010/0177368 A1 ein Endoskop mit einem Lichtsender, einem Aktuator und einem Kraftübertrager. Der Lichtsender, beispielsweise einer Faser, erstreckt sich in einer ersten Richtung. Der Aktuator biegt den Lichtsender in einer zweiten Richtung durch Drücken einer Seite des Lichtsenders in die zweite Richtung. Die zweite Richtung ist senkrecht zu der ersten Richtung. Der Aktuator umfasst einen Biegeblock, welcher durch piezoelektrische Elemente gebildet wird.

Die US 2014/0354790 A1 betrifft ein Endoskop mit einer Faser, einem ersten Aktuator, welcher seitlich zu der Faser angeordnet ist, um die Faser zu schwenken, einen zweiten Aktuator, welcher an einer Position gegenüberliegend zu dem ersten Aktuator angeordnet ist, wobei die Faser dazwischen angeordnet ist, um die Faser zu schwenken, und einen Signalgenerator, um Steuersignale zum Schwenken des Endabschnitts der Faser zu erzeugen.

Die US2017/0010461 und die entsprechende WO 2015/151593A1 betreffen einen Lichtwellenleiterabtaster, welcher einen länglichen Lichtwellenleiter, piezoelektrische Antriebselemente, die auf einer Außenumfangsfläche des Lichtwellenleiters bereitgestellt sind und eine Biegungsschwingung an einem distalen Endabschnitt des Lichtwellenleiters infolge dessen erzeugen, dass eine Wechselspannung daran angelegt wird, piezoelektrische Detektionselemente, die auf der Außenumfangsfläche am distalen Endabschnitt des Lichtwellenleiters bereitgestellt sind, und eine Steuereinheit, die eine Wechselspannung, die an die piezoelektrischen Antriebselemente angelegt wird, auf Basis der Spannungen steuert, die von den piezoelektrischen Detektionselementen erzeugt werden.

Solche Techniken weisen jedoch bestimmte Nachteile und Einschränkungen auf. Zum Beispiel kann eine Amplitude der Bewegung bei einer entsprechenden Geometrie der Anregung vergleichsweise limitiert sein. Dies kann für optische Anwendungen den Sichtbereich einschränken. Ferner können die Freiheitsgrade der Bewegung die mittels einer entsprechenden Geometrie der Anregung angeregt werden können, beschränkt sein.

### KURZE ZUSAMMENFASSUNG DER ERFINDUNG

Deshalb besteht ein Bedarf für verbesserte Techniken zur Anregung einer Faser. Insbesondere besteht ein Bedarf für solche Techniken, die zumindest einige der oben genannten Nachteile und Einschränkungen lindern oder beheben.

Diese Aufgabe wird von den Merkmalen der unabhängigen Patentansprüche gelöst. Die Merkmale der abhängigen Patentansprüche definieren Ausführungsformen.

In einem Beispiel umfasst eine Vorrichtung zur Anregung einer Faser einen ersten Biegepiezoaktuator und einen zweiten Biegepiezoaktuator. Die Vorrichtung umfasst auch ein Verbindungsteil. Das Verbindungsteil ist zwischen dem ersten Biegepiezoaktuator und dem zweiten Biegepiezoaktuator angeordnet. Die Vorrichtung umfasst auch eine bewegliche Faser, die am Verbindungsteil angebracht ist.

In einem weiteren Beispiel umfasst ein Verfahren das Ansteuern eines ersten Biegepiezoaktuators mit einer ersten Signalform und das Ansteuern eines zweiten Biegepiezoaktuators mit einer zweiten Signalform. Dadurch wird erreicht, dass eine bewegliche Faser, die an einem Verbindungsteil, welches zwischen dem ersten Biegepiezoaktuator und dem zweiten Biegepiezoaktuator angeordnet ist, angeregt wird.

In einem weiteren Beispiel umfasst ein System ein längliches erstes Gehäuse und ein längliches zweites Gehäuse. Das längliche zweite Gehäuse erstreckt sich zumindest in einem Abschnitt entlang des ersten Gehäuses. Das System umfasst auch eine Hochfrequenz-Antenne. Die Hochfrequenz-Antenne ist in dem ersten Gehäuse angeordnet. Das System umfasst auch eine Vorrichtung mit einem ersten Biegepiezoaktuator und einem zweiten Biegepiezoaktuator, einem Verbindungsteil und einer beweglichen Faser gemäß verschiedener anderer hierin beschriebener Beispiele.

Solche Beispiele können in verschiedenen weiteren Beispielen miteinander kombiniert werden.

### KURZE BESCHREIBUNG DER FIGUREN

FIG. 1 illustriert schematisch eine Vorrichtung zur Anregung einer Faser, die zwei Biegepiezoaktuatoren gemäß verschiedener Ausführungsformen aufweist.
FIG. 2 illustriert schematisch einen Biegepiezoaktuator gemäß verschiedener Ausführungsformen.
FIG. 3 illustriert schematisch eine Vorrichtung zur Anregung einer Faser, die zwei Biegepiezoaktuatoren gemäß verschiedener Ausführungsformen aufweist.
FIG. 4 illustriert schematisch eine Vorrichtung gemäß verschiedener Ausführungsformen, die einen Treiber für mindestens einen Aktuator und mindestens einen Aktuator wie beispielsweise einen Biegepiezoaktuator aufweist.
FIG. 5 ist ein Flussdiagramm eines Verfahrens gemäß verschiedener Ausführungsformen.
FIG. 6 illustriert schematisch Signalbeiträge von Signalformen mit denen ein Treiber Biegepiezoaktuatoren gemäß verschiedener Ausführungsformen ansteuert, wobei die Signalbeiträge gegenphasig ausgebildet sind.
FIG. 7 illustriert schematisch Signalbeiträge von Signalformen mit denen ein Treiber Biegepiezoaktuatoren gemäß verschiedener Ausführungsformen ansteuert, wobei die Signalbeiträge gleichphasig ausgebildet sind.
FIG. 8 illustriert schematisch Signalbeiträge von Signalformen mit denen ein Treiber Biegepiezoaktuatoren gemäß verschiedener Ausführungsformen ansteuert, wobei die Signalbeiträge gegenphasig ausgebildet sind und einen DC-Anteil aufweisen.
FIG. 9 illustriert schematisch Signalbeiträge von Signalformen mit denen ein Treiber Biegepiezoaktuatoren gemäß verschiedener Ausführungsformen ansteuert, wobei die Signalbeiträge gleichphasig ausgebildet sind und einen DC-Anteil aufweisen.
FIG. 10 illustriert schematisch eine Vorrichtung zur Anregung einer Faser, die zwei Biegepiezoaktuatoren und eine Magnetfeldquelle gemäß verschiedener Ausführungsformen aufweist.
FIG. 11 ist eine schematische Aufsicht auf ein Verbindungsteil, welches gemäß verschiedener Ausführungsformen zwischen zwei Biegepiezoaktuatoren angeordnet ist.
FIG. 12 ist eine schematische Seitenansicht des Verbindungsteil gemäß FIG. 11.
FIG. 13 ist eine schematische Aufsicht auf ein Verbindungsteil, welches gemäß verschiedener Ausführungsformen zwischen zwei Biegepiezoaktuatoren angeordnet ist.
FIG. 14 ist eine schematische Seitenansicht des Verbindungsteil gemäß FIG. 13.
FIG. 15 illustriert schematisch eine Vorrichtung zur Anregung einer Faser, die zwei Biegepiezoaktuatoren gemäß verschiedener Ausführungsformen aufweist.
FIG. 16 illustriert schematisch ein System gemäß verschiedener Ausführungsformen, welches ein erstes Gehäuse mit einer Hochfrequenz-Antenne und ein zweites Gehäuse mit einer Vorrichtung zur Anregung einer Faser gemäß verschiedener Beispiele aufweist.

### DETAILLIERTE BESCHREIBUNG VON AUSFÜHRUNGSFORMEN

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionale Einheiten können als Hardware, Software oder eine Kombination aus Hardware und Software implementiert werden.

Nachfolgend werden Techniken zur Anregung eines faserförmigen Elements beschrieben (das faserförmige Element wird nachfolgend aus Gründen der Knappheit einfach als Faser bezeichnet). Die Anregung der Faser bewirkt eine Bewegung der Faser. In den verschiedenen hierin beschriebenen Beispielen können unterschiedliche Bewegungen bzw. Freiheitsgrade der Bewegung der Faser angeregt werden. Beispiele umfassen eine Torsionsmode, bei der die Faser entlang ihrer Längsachse eine Verdrillung erfährt. Weitere Beispiele umfassen eine oder mehrere Transversalmoden, bei denen die Faser senkrecht zu ihrer Längsachse ausgelenkt wird. In manchen Beispielen ist es möglich, dass Transversalmoden unterschiedlicher Orientierung - zum Beispiel senkrecht zueinander - angeregt werden, d.h. orthogonale Transversalmoden. In manchen Beispielen ist es möglich, dass Transversalmoden unterschiedlicher Ordnung, zum Beispiel erster Ordnung oder zweiter Ordnung, angeregt werden. Die Transversalmoden unterschiedlicher Ordnung können eine unterschiedliche Anzahl von Knoten und Bäuchen aufweisen.

Es können unterschiedliche Typen von Fasern verwendet werden. Beispielsweise können Lichtfasern verwendet werden, die auch als Glasfasern bezeichnet werden. Dies ist aber nicht erforderlich. Dabei ist es aber nicht erforderlich, dass die Fasern aus Glas hergestellt sind. Die Fasern können zum Beispiel aus Kunststoff, Glas, Silizium, oder einem anderen Material hergestellt sein. Beispielsweise können die Fasern aus Quarzglas hergestellt sein. Beispielsweise können die Fasern ein 70 GPa Elastizität-Modul aufweisen oder ein Elastizität-Modul im Bereich von 40 GPa - 80 GPa, vorzugsweise im Bereich 60 - 75 GPa.

Beispielsweise können die Fasern ein Elastizität-Modul im Bereich von 140 GPa - 200 Gpa aufweisen. Beispielsweise können die Fasern bis zu 4 % Materialdehnung ermöglichen. In manchen Beispielen weisen die Fasern einen Kern auf, in welchem das eingespeiste Laserlicht propagiert und durch Totalreflektion an den Rändern eingeschlossen ist (Lichtwellenleiter). Die Faser muss aber keinen Kern aufweisen. In verschiedenen Beispielen können sogenannte Einmoden-Lichtfasern (engl. single mode fibers) oder Mehrmoden-Lichtfasern (engl. multimode fibers) verwendet werden. Die verschiedenen hierin beschriebenen Fasern können zum Beispiel einen kreisförmigen Querschnitt aufweisen. Es wäre zum Beispiel möglich, dass die verschiedenen hierin beschriebenen Fasern einen Durchmesser aufweisen, der nicht kleiner als 50 µm ist, optional nicht <150 µm ist, weiter optional nicht <500 µm ist, weiter optional nicht <1 mm ist. Der Durchmesser kann aber auch < 1 mm sein, optional < 500 µm, weiter optional kleiner 150 µm. Zum Beispiel können die verschiedenen hierin beschriebenen Fasern verbiegbar bzw. krümmbar ausgestaltet sein, d.h. flexibel. Dazu kann das Material der hierin beschriebenen Fasern eine gewisse Elastizität aufweisen. Deshalb können die Fasern auch als Federelemente bezeichnet werden. Die Fasern können z.B. eine Länge im Bereich von 3 mm bis 12 mm aufweisen, optional im Bereich von 4 mm bis 8 mm.

Die angeregte Faser kann in unterschiedlichsten Anwendungsgebieten eingesetzt werden. Beispiele umfassen zum Beispiel die Entfernungsmessung mittels gepulstem Laserlicht (auch als engl. Light detection and ranging, LIDAR bezeichnet; manchmal auch LADAR). In einem solchen Fall wäre es möglich, dass das Laserlicht durch einen Lichtwellenleiter im Bereich des Kerns der Faser geleitet wird und am beweglichen Ende der Faser aus der Faser austritt. Es wäre aber alternativ oder zusätzlich auch möglich, dass das Laserlicht nicht durch die Faser verläuft und zum Beispiel von einer Umlenkeinheit - wie einem Prisma oder einem Spiegel -, die am beweglichen Ende der Faser angebracht ist, umgelenkt wird. Z.B. kann eine Rückseitenbefestigung eines Spiegels verwendet werden: dabei kann sich die Faser von einer der Spiegelfläche gegenüberliegenden Rückseite des Spiegels wegerstrecken. Damit kann es möglich sein, das gepulste Laserlicht zu scannen. Ein weiteres Anwendungsgebiet wäre zum Beispiel die Endoskopie. Ein weiteres Anwendungsgebiet wäre zum Beispiel ein Projektor, der sichtbares Licht mit unterschiedlichen Farben, wie beispielsweise rot, grün und blau scannt.

Nachfolgend werden verschiedene Techniken zum Scannen von Licht beschrieben. Die nachfolgend beschriebenen Techniken können zum Beispiel das eindimensionale oder zweidimensionale Scannen von Licht ermöglichen. Das Scannen kann wiederholtes Aussenden des Lichts unter unterschiedlichen Abstrahlwinkeln bzw. Winkelbereichen bezeichnen. Das wiederholte Umsetzen eines bestimmten Winkelbereichs kann eine Wiederholrate des Scannens bestimmen. Die Menge der Winkelbereiche kann einen Scanbereich bzw. einen Bildbereich definieren. Das Scannen kann das wiederholte Abtasten von unterschiedlichen Scanpunkten in der Umgebung mittels des Licht bezeichnen. Für jeden Scanpunkt können Messsignale ermittelt werden.

In manchen Beispielen wird ein eindimensionaler Scanbereich implementiert. Dazu kann es zum Beispiel möglich sein, gezielt einen einzelnen Freiheitsgrad der Bewegung der Faser anzuregen. In weiteren Beispielen kann aber auch ein zweidimensionaler Scanbereich implementiert werden. Dazu kann es zum Beispiel möglich sein, einen ersten Freiheitsgrad der Bewegung der Faser überlagert mit einem zweiten Freiheitsgrad der Bewegung der Faser anzuregen. Dies bedeutet, dass die Faser zeitlich und örtlich überlagerte Bewegungen ausführen kann. Beispielsweise wäre es möglich, eine Transversalmode der Faser überlagert mit einer Torsionsmode der Faser anzuregen. Beispielsweise könnte die Transversalmode erster Ordnung und/oder zweiter Ordnung der Faser überlagert mit einer Torsionsmode der Faser angeregt werden.

In verschiedenen Beispielen kann die Faser resonant angeregt werden, das heißt bei oder nahe bei einer Resonanzfrequenz. Beispielsweise kann die Faser semi-resonant angeregt werden, d.h. in der Flanke einer Resonanzkurve.

In verschiedenen Beispielen können zur Anregung der Faser Biegepiezoaktuatoren verwendet werden. Zum Beispiel können ein erster und ein zweiter Biegepiezoaktuator verwendet werden. Es wäre möglich, dass der erste Biegepiezoaktuator und/oder der zweite Biegepiezoaktuator plattenförmig ausgebildet sind. Im Allgemeinen kann eine Dicke der Biegepiezoaktuatoren z.B. im Bereich von 200 µm - 1 mm liegen, optional im Bereich von 300 µm - 700 µm. Es wäre beispielsweise möglich, dass der erste Biegepiezoaktuator und/oder der zweite Biegepiezoaktuator eine Schichtstruktur umfassend eine alternierende Anordnung mehrerer piezoelektrischer Materialien aufweist. Diese können einen unterschiedlich starken piezoelektrischen Effekt aufweisen. Dadurch kann eine Verbiegung bewirkt werden, ähnlich einem Bimetallstreifen bei Temperaturänderungen. Beispielsweise ist es möglich, dass der erste Biegepiezoaktuator und/oder der zweite Biegepiezoaktuator an einer Fixierstelle fixiert sind: ein der Fixierstelle gegenüberliegendes Ende kann dann aufgrund einer Verbiegung bzw. Krümmung des ersten Biegepiezoaktuators und/oder des zweiten Biegepiezoaktuators bewegt werden.

Durch die Verwendung von Biegepiezoaktuatoren kann eine besonders effiziente und starke Anregung der Faser erreicht werden. Außerdem kann es möglich sein, eine hohe Integration der Vorrichtung zur Anregung der Faser zu erzielen. Dies kann bedeuten, dass der benötigte Bauraum besonders gering dimensioniert werden kann.

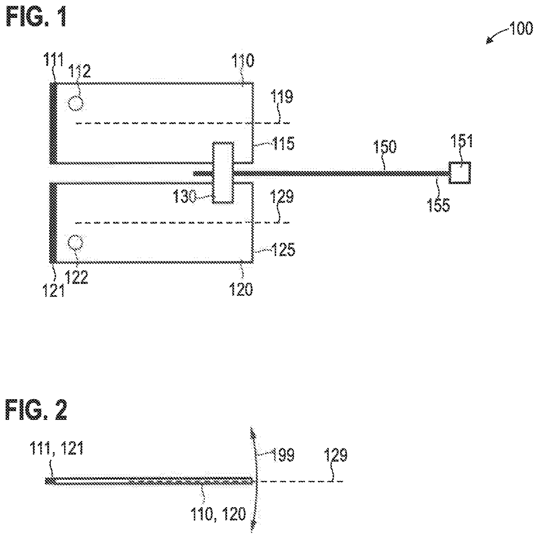

FIG. 1 illustriert Aspekte in Bezug auf eine Vorrichtung 100. Insbesondere illustriert FIG. 1 Aspekte in Bezug auf eine Anordnung von Piezoaktuatoren 110, 120 in Bezug auf eine Faser 150. In dem Beispiel der FIG. 1 sind die Piezoaktuatoren 110, 120 als Biegepiezoaktuatoren ausgebildet. Dies bedeutet, dass das Anlegen einer Spannung an elektrischen Kontakten 112, 122 der Biegepiezoaktuatoren 110, 120 eine Krümmung bzw. Verbiegung der Biegepiezoaktuatoren 110, 120 entlang deren Längsachse 119, 129 bewirkt. Dazu weisen die Biegepiezoaktuatoren 110, 120 eine Schichtstruktur auf (in FIG. 1 nicht dargestellt und senkrecht zur Zeichenebene orientiert). Derart wird ein Ende 115, 125 der Biegepiezoaktuatoren 110, 120 gegenüber einer Fixierstelle 111, 121 senkrecht zur jeweiligen Längsachse 119, 129 ausgelenkt (die Auslenkung ist in dem Beispiel der FIG. 1 senkrecht zur Zeichenebene orientiert). Die Auslenkung 199 der Biegepiezoaktuatoren 110, 120 aufgrund der Verbiegung ist in FIG. 2 dargestellt. FIG. 2 ist eine Seitenansicht der Biegepiezoaktuatoren 110, 120. FIG. 2 zeigt die Biegepiezoaktuatoren 110, 120 in einer Ruhelage, z.B. ohne Treiber-Signal bzw. Verspannung / Krümmung.

Beispielsweise könnten die Fixierstellen 111, 121 eine starre Verbindung zwischen den Biegepiezoaktuatoren 110, 120 und einem Gehäuse der Vorrichtung 100 (in FIG. 1 nicht dargestellt) herstellen.

In dem Beispiel der FIG. 1 umfasst die Vorrichtung 100 auch ein Verbindungsteil 130. Das Verbindungsteil 130 ist zwischen den Biegepiezoaktuatoren 110, 120 in einem Bereich angrenzend an die beweglichen Enden 115, 125 angeordnet. Dadurch bewirkt eine Auslenkung 199 der Biegepiezoaktuatoren 110, 120 auch eine Bewegung des Verbindungsteil 130.

Das Verbindungsteil 130 ist auch mit der Faser 150 verbunden, beispielsweise durch Kleber. Das Verbindungsteil 130 könnte auch einstückig mit der Faser 150 ausgebildet sein. Dadurch überträgt sich eine Bewegung der Biegepiezoaktuatoren 110, 120 über das Verbindungsteil 130 auf die Faser 150. Die Faser 150 erstreckt sich weg vom Verbindungsteil 130. Dadurch kann die Faser 150 angeregt werden. Beispielsweise könnte eine Transversalmode und/oder eine Torsionsmode der Faser 150 angeregt werden. Dadurch wird insbesondere bewirkt, dass sich ein bewegliches Ende 155 der Faser 150, welches gegenüberliegend zu dem Verbindungsteil 130 angeordnet ist, bewegen kann. Zum Beispiel kann eine Krümmung oder Verdrehung der Faser 150 im Bereich des beweglichen Endes 150 durch eine Transversalmode bzw. eine Torsionsmode erreicht werden.

Die Faser kann auch über das Verbindungsteil 130 in eine Richtung entfernt vom beweglichen Ende hinaus fortgeführt werden (in FIG. 1 nur ansatzweise gezeigt). Dann kann z.B. Licht in die Faser eingespeist werden und zum beweglichen Ende 155 hin geführt werden.

Die Vorrichtung 100 ermöglicht ein besonders effizientes Anregen der Faser 150. Beispielsweise können Bewegungen der Faser 150 mit einer besonders großen Amplitude angeregt werden. Es wurde darüber hinaus beobachtet, dass mittels der Vorrichtung 100 die Torsionsmode der Faser 150 besonders effizient angeregt werden kann.

Am beweglichen Ende 155 der Faser 150 ist auch ein optisches Element 151 angebracht. Das optische Element 151 kann die Bewegung der Faser 155 übersetzen in eine Umlenkung von Licht, beispielsweise von Laserlicht. Dadurch kann das Licht gescannt werden. In manchen Beispielen kann das Licht entlang der Längsachse durch die Faser 150 verlaufen, beispielsweise in einem Lichtwellenleiter nahe des Kerns der Faser 150. In anderen Beispielen könnte das Licht jedoch auch auf einem anderen Weg zum optischen Element 151 gelangen. Beispielsweise kann das optische Element 151 eine Linse, wie beispielsweise eine GRIN-Linse (engl. graded index lense), aufweisen. Beispielsweise wäre es möglich, dass das optische Element 151 eine Umlenkeinheit, beispielsweise einen Spiegel oder ein Prisma, aufweist.

In dem Beispiel der FIG. 1 ist die Längsachse 119 des Biegepiezoaktuators 110 parallel zu der Längsachse 129 des Biegepiezoaktuators 120. Dies bedeutet, dass die Längsachsen 119, 129 einen Winkel von ca. 0° miteinander einschließen. Im Allgemeinen ist es möglich, dass die Längsachsen 119, 129 einen vergleichsweise kleinen Winkel miteinander einschließen, d. h. in etwa parallel zueinander verlaufen. Beispielsweise wäre es möglich, dass die Längsachsen 119, 129 einen Winkel miteinander einschließen, der kleiner als 20° ist, optional kleiner als 10°, weiter optional kleiner als 1°. Durch eine solche parallele Konfiguration der Biegepiezoaktuatoren 110, 120 kann eine Bewegung der Biegepiezoaktuatoren 110, 120 besonders effizient in eine Bewegung der Faser 150 übersetzt werden. Außerdem ist es möglich, dass der von der Vorrichtung 100 benötigte Bauraum besonders gering dimensioniert ist. Dies kann zum Beispiel insbesondere in Bezug auf Referenzimplementierungen zutreffen, bei welchen die Biegepiezoaktuatoren einen vergleichsweise großen Winkel miteinander einschließen.

In dem Beispiel der FIG. 1 ist ferner die Längsachse 119 parallel zur Längsachse der Faser 150. Außerdem ist die Längsachse 129 parallel zur Längsachse der Faser 150. Im Allgemeinen ist es möglich, dass die Längsachsen 119, 129 einen vergleichsweise kleinen Winkel mit der Längsachse der Faser 150 einschließen, d. h. in etwa parallel zur Längsachse der Faser 150 verlaufen. Beispielsweise wäre es möglich, dass die Längsachse 119 und/oder die Längsachse 129 einen Winkel mit der Längsachse der Faser 150 einschließt, der kleiner als 20° ist, optional kleiner als 10°, weiter optional kleiner als 1°. Eine solche parallele Anordnung der Biegepiezoaktuatoren 110, 120 zur Faser 150 ermöglicht eine hohe Integration der Vorrichtung 100. Der Bauraum kann besonders gering dimensioniert werden. Dies trifft insbesondere zu in Bezug auf Referenzimplementierungen, bei welchen ein hierzu Aktuator senkrecht zur Faser 150 orientiert ist.

In dem Beispiel der FIG. 1 ist eine Konfiguration gezeigt, in welcher sich die Biegepiezoaktuatoren 110, 120 entlang ihrer Längsachse 119, 129 weg vom beweglichen Ende 155 der Faser 150 erstrecken. Dies bedeutet, dass der Bereich der Auslenkung der Faser 150 entfernt von den Biegepiezoaktuatoren 110, 120 angeordnet ist. Es werden aber auch andere Konfigurationen denkbar.

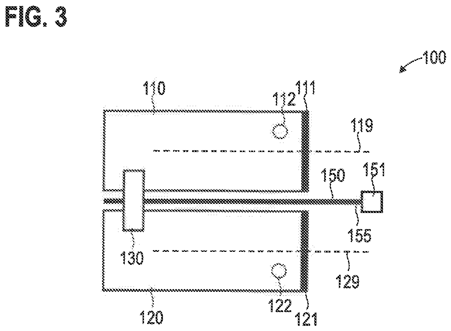

FIG. 3 illustriert Aspekte in Bezug auf eine Vorrichtung 100. Insbesondere illustriert FIG. 3 Aspekte in Bezug auf eine Anordnung von Biegepiezoaktuatoren 110, 120 in Bezug auf eine Faser 150. das Beispiel der FIG. 3 entspricht grundsätzlich dem Beispiel der FIG. 1.in dem Beispiel der FIG. 3 erstrecken sich die Biegepiezoaktuatoren 110, 120 jedoch entlang ihrer Längsachsen 119, 129 hin zum beweglichen Ende 155 der Faser 150. Dadurch kann eine besonders hohe Integration der Vorrichtung 100 erreicht werden.

Beispielsweise ist aus einem Vergleich der FIGs. 1 und zwei ersichtlich, dass sich in dem Beispiel der FIG. 1 das Verbindungsteil 130 zwischen den Fixierstellen 111, 121 und dem beweglichen Ende 155 der Faser 150 befindet - in dem Beispiel der FIG. 3 jedoch die Fixierstellen 111, 121 zwischen dem Verbindungsteil 130 und dem beweglichen Ende 155 angeordnet sind.

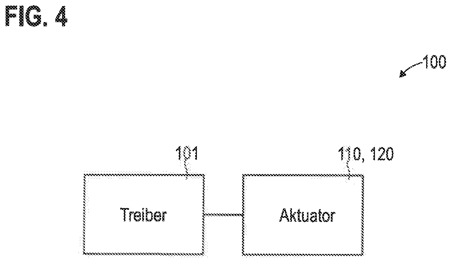

FIG. 4 illustriert Aspekte in Bezug auf die Vorrichtung 100. Insbesondere illustriert FIG. 4 Aspekte in Bezug auf einen Treiber 101 der Bewegung. Beispielsweise könnte der Treiber 101 ein oder mehrere Treiberschaltungen umfassen. Beispielsweise könnte der Treiber 101 eingerichtet sein, um ein Treiber-Signal mit einer bestimmten Signalform an ein oder mehrere Aktuatoren - beispielsweise die Biegepiezoaktuatoren 110, 120 und/oder einen Magnet-Aktuator - auszugeben. Der Treiber 101 kann analoge Komponenten und/oder digitale Komponenten aufweisen. Beispielsweise könnte der Treiber 101 eingerichtet sein, um die Aktuatoren in Abhängigkeit von einem digitalen Steuersignal anzusteuern. Da Treiber-Signal kann bestimmte Signalformen aufweisen, die zur Ansteuerung der verschiedenen Aktuatoren verwendet werden. Das Treiber-Signal ist typischerweise ein analoges Signal.

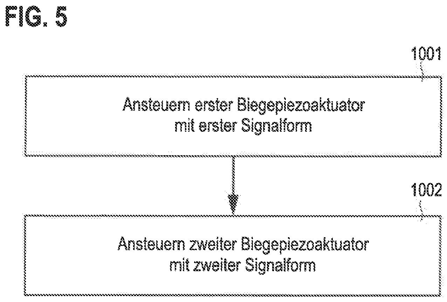

FIG. 5 ist ein Flussdiagramm eines Verfahrens gemäß verschiedener Beispiele. Beispielsweise könnte das Verfahren gemäß FIG. 5 von dem Treiber 101 ausgeführt werden.

Zunächst wird in Block 1001 der erste Biegepiezoaktuator 110 mit einer ersten Signalform angesteuert. Dies kann zum Beispiel das Bereitstellen einer Spannung und/oder eines Stromflusses umfassen.

Dann wird in Block 1002 der zweite Biegepiezoaktuator 120 mit einer zweiten Signalform angesteuert. Dies kann zum Beispiel das Bereitstellen einer Spannung und/oder eines Stromflusses umfassen.

Im Allgemeinen ist es möglich, dass die verschiedenen Aktuatoren- wie beispielsweise die Biegepiezoaktuatoren 110, 120 - zumindest teilweise zeitparallel angesteuert werden. Insbesondere wäre es möglich, dass die verschiedenen Aktuatoren Phasen-kohärent angesteuert werden. Dies kann bedeuten, dass die verschiedenen Signalformen zum Ansteuern der verschiedenen Aktuatoren eine wohldefinierte Phasenbeziehung zueinander aufweisen.

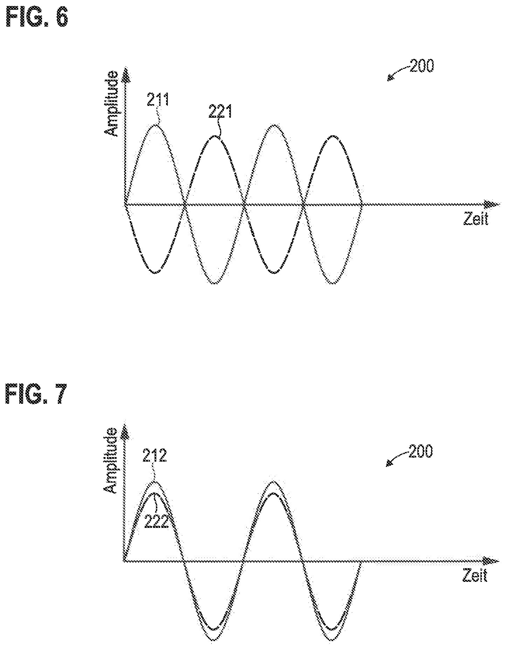

FIG. 6 illustriert Aspekte in Bezug auf Signalformen 200, die dazu verwendet werden können, um die Biegepiezoaktuatoren 110, 120 gemäß verschiedener hierin beschriebener Beispiele anzusteuern. FIG. 6 illustriert insbesondere den Verlauf der Amplitude der Signalformen 200 als Funktion der Zeit.

In dem Beispiel der FIG. 6 ist ein Signalbeitrag 211 (durchgezogene Linie) dargestellt, der dazu verwendet wird, um den Biegepiezoaktuator 110 anzusteuern. Außerdem ist in dem Beispiel der FIG. 6 ein Signalbeitrag 221 (gestrichelte Linie) dargestellt, der dazu verwendet wird, um den Biegepiezoaktuator 120 anzusteuern. Aus dem Beispiel der FIG. 6 ist ersichtlich, dass die Signalbeiträge 211, 221 gegenphasig konfiguriert sind. Dies bedeutet im Beispiel der FIG. 6, dass die Signalbeiträge 211, 221 dieselbe Frequenz aufweisen, sowie einen Phasenversatz von 180°.

Dadurch kann erreicht werden, dass sich der Biegepiezoaktuator 110 nach oben krümmt bzw. bewegt (nach unten krümmt bzw. bewegt), während sich der Biegepiezoaktuator 120 nach unten krümmt bzw. bewegt (nach oben krümmt bzw. bewegt). Dadurch kann wiederum erreicht werden, dass das Verbindungsteil 130 abwechselnd nach links und nach rechts verkippt wird (in Bezug auf die Längsachse der Faser 150). Deshalb kann mit einer solchen Konfiguration der Signalformen 200 eine besonders effiziente Anregung der Torsionsmode der Faser 150 erzielt werden.

FIG. 7 illustriert Aspekte in Bezug auf Signalformen 200, die dazu verwendet werden können, um die Biegepiezoaktuatoren 110, 120 gemäß verschiedener hierin beschriebener Beispiele anzusteuern. FIG. 7 illustriert insbesondere den Verlauf der Amplitude der Signalformen 200 als Funktion der Zeit.

In dem Beispiel der FIG. 7 ist ein Signalbeitrag 212 (durchgezogene Linie) dargestellt, der dazu verwendet wird, um den Biegepiezoaktuator 110 anzusteuern. Außerdem ist in dem Beispiel der FIG. 7 ein Signalbeitrag 222 (gestrichelte Linie) dargestellt, der dazu verwendet wird, um den Biegepiezoaktuator 120 anzusteuern. Aus dem Beispiel der FIG. 7 ist ersichtlich, dass die Signalbeiträge 212, 222 gleichphasig konfiguriert sind. Dies bedeutet im Beispiel der FIG. 7, dass die Signalbeiträge 212, 222 dieselbe Frequenz aufweisen, sowie einen Phasenversatz von 0°. In manchen Beispielen wäre es möglich, dass die gleichphasigen Signalbeiträge 212, 222 eine Amplitudenmodulation aufweisen.

Durch die gleichphasigen Signalbeiträge 212, 222 kann erreicht werden, dass sich der Biegepiezoaktuator 110 nach oben krümmt bzw. bewegt (nach unten krümmt bzw. bewegt), während sich der Biegepiezoaktuator 120 nach oben krümmt bzw. bewegt (nach unten krümmt bzw. bewegt). Dadurch kann wiederum erreicht werden, dass das Verbindungsteil 130 abwechselnd nach oben und unten bewegt wird (in Bezug auf die Längsachse der Faser 150). Deshalb kann mit einer solchen Konfiguration der Signalformen 200 eine besonders effiziente Anregung von Transversalmode in der Faser 150 erzielt werden.

Eine typische Frequenz der Signalbeiträge 211, 212, 221, 222 in den verschiedenen hierin beschriebenen Beispielen liegt zum Beispiel im Bereich von 50 Hz-1,5 kHz, optional im Bereich von 200 Hz-1 kHz, weiter optional im Bereich von 500 Hz-700 Hz. Derart können angemessene Bildwiederholfrequenz in erzielt werden.

In den Beispielen der FIGs. 6 und 7 sind Szenarien illustriert, in welchen die gegenphasige Signalbeiträge 211, 221 zum Anregen der Biegepiezoaktuatoren 110, 120 in etwa dieselbe Frequenz aufweisen, wie die gleichphasige Signalbeiträge 212, 222 zum Anregen der Biegepiezoaktuatoren 110, 120. Im Allgemeinen wäre es möglich, dass die gegenphasige Signalbeiträge 211, 221 eine erste Frequenz im Bereich von 95 - 105 % einer zweiten Frequenz der gleichphasige Signalbeiträge 212, 222 aufweisen. Mittels einer solchen Implementierung der Frequenzen der Signalformen 200 kann erreicht werden, dass eine besonders effiziente Überlagerungsfigur der verschiedenen Freiheitsgrade der Bewegung der Faser 150 - zum Beispiel der Torsionsmode mit der Transversalmode - erzielt werden kann. Insbesondere kann dadurch erreicht werden, dass eine hohe Bildwiederholrate erzielt werden kann, ohne dass bestimmte Bereiche eines Scanbereich durch Knoten in der Überlagerungsfigur mehrfach gescannt werden. Insbesondere können solche Implementierungen der Frequenzen der Signalformen 200 ausnutzen, dass eine Entartung der verschiedenen angeregten Freiheitsgrade der Bewegung der Faser 150 im Frequenzraum vorliegt. Zum Beispiel kann es möglich sein, eine Entartung der Frequenz der Torsionsmode der Faser 150 und der Frequenz der Transversalmode der Faser 150 durch geeignetes Konfigurieren ein oder mehrere der folgenden Parameter zu erreichen: Länge der Faser 150; Massenträgheitsmoment der Faser 150 und/oder eines Wuchtgewichts, dass an der Faser 150 angebracht ist; und Massenträgheitsmoment des optischen Elements 151.

In anderen Beispielen wäre es jedoch auch möglich, dass die gegenphasige Signalbeiträge 211, 221 eine andere erste Frequenz aufweisen, als die zweite Frequenz der gleichphasigen Signalbeiträge 212, 222. Beispielsweise könnte die erste Frequenz der gegenphasige Signalbeiträge 211, 221 im Bereich von 45-55 % der zweiten Frequenz der gleichphasigen Signalbeiträge 212, 222 liegen, d. h. in etwa die Hälfte der zweiten Frequenz betragen. In anderen Beispielen könnte die erste Frequenz auch in etwa doppelt so groß sein wie die zweite Frequenz oder einen ganz anderen Wert annehmen. Durch eine solche Aufhebung der Entartung zwischen den verschiedenen durch die gegenphasigen Signalbeiträge 211, 221 und gleichphasigen Signalbeiträge 212, 222 angeregten Freiheitsgrade der Bewegung der Faser 150 können nichtlineare Wechselwirkungen zwischen den entsprechenden Freiheitsgraden der Bewegung vermieden werden. Zum Beispiel kann das Ausbilden eines parametrischen Oszillators durch die Transversalmode und/oder die Torsionsmode vermieden werden. Dadurch kann ein besonders gezieltes Anregen der Faser 150 erreicht werden.

Aus einer Überlagerung der gleichphasigen Signalbeiträge 211, 221 mit den gegenphasigen Signalbeiträgen 212, 222 kann erreicht werden, dass die Signalform 200 am Biegepiezoaktuator 110 eine bestimmte Phasenverschiebung gegenüber der Signalform 200 am Biegepiezoaktuator 120 aufweist. Diese Phasenverschiebung kann variiert werden, z.B. in Abhängigkeit der relativen Amplitude der gleichphasigen Signalbeiträge 211, 221 und gegenphasigen Signalbeiträge 212, 222 zueinander. In anderen Worten können die tatsächlichen Signalformen 200 zerlegt werden in die gleichphasigen Signalbeiträge 211, 221 und die gegenphasigen Signalbeiträge 212, 222. In manchen Beispielen kann ein zur Erzeugung der Signalformen 200 verwendeter Funktionsgenerator in bereits die Überlagerung der gleichphasigen Signalbeiträge 211, 221 mit den gegenphasigen Signalbeiträgen 212, 222 erzeugen kann.

In manchen hierein beschriebenen Beispielen kann es erstrebenswert sein, durch die Torsionsmode eine große Amplitude der Verdrehung der Umlenkeinheit 151 zu erzielen, um Licht zu scannen. Z.B. kann die Amplitude der Verdrehung der Umlenkeinheit 151 auf einen bestimmten Sollwert geregelt werden, der größer als Null ist und z.B. im Bereich von 10° - 120° liegt. Andererseits kann durch gezieltes Verwenden der gleichphasigen Signalbeiträge 211, 221 die ungewollte Anregung der Transversalmode durch externen Schock aktiv gedämpft werden. Dazu könnte z.B. ein Positionssensor vorhanden sein, der die transversale Auslenkung der Umlenkeinheit 151 misst; dann kann basierend auf dem Messsignal des Positionssensors die Amplitude und/oder Phase der gleichphasigen Signalbeiträge 211, 221 eingestellt werden; z.B. könnte eine Regelschleife implementiert werden, welche als Sollgröße eine minimale transversale Auslenkung der Umlenkeinheit 151 vorgibt. Alternativ oder zusätzlich zum Messen der transversalen Auslenkung der Umlenkeinheit 151 könnte auch ein Beschleunigungssensor im Referenzkoordinatensystem der unbewegten Enden 111, 121 angeordnet sein und den externen Schock messen.

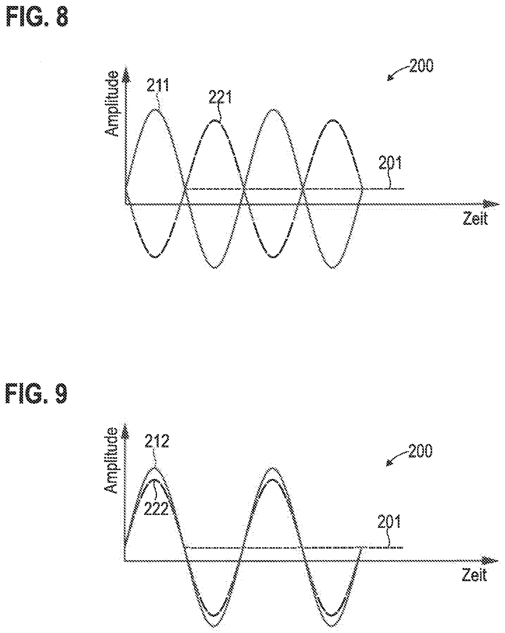

FIG. 8 illustriert Aspekte in Bezug auf Signalformen 200, die dazu verwendet werden können, um die Biegepiezoaktuatoren 110, 120 gemäß verschiedener hierin beschriebener Beispiele anzusteuern. FIG. 8 illustriert insbesondere den Verlauf der Amplitude der Signalformen 200 als Funktion der Zeit.

Das Beispiel der FIG. 8 entspricht grundsätzlich dem Beispiel der FIG. 6.jedoch weisen in dem Beispiel der FIG. 8 die Signalbeiträge 211, 221 jeweils einen DC-Anteil 201 auf. In manchen Beispielen wäre es auch möglich, dass lediglich einer der Signalbeiträge 211, 221 einen DC-Anteil 201 (horizontale gestrichelte Linie in FIG. 8) aufweist. In manchen Beispielen wäre es auch möglich, dass die beiden Signalbeiträge 211,221 unterschiedlich dimensionierte DC-Anteile 201 aufweisen, zum Beispiel in Größe und/oder Vorzeichen.

Durch das Vorsehen des DC-Anteils 201 kann erreicht werden, dass eine Vorspannung (engl. bias) der Faser 150, d. h. eine DC-Auslenkung der Faser 150, implementiert wird. Dadurch können zum Beispiel ein Versatz der Faser und/oder Vorgaben für das Sichtfeld des entsprechenden Scanners kompensiert oder berücksichtigt werden.

FIG. 9 illustriert Aspekte in Bezug auf Signalformen 200, die dazu verwendet werden können, um die Biegepiezoaktuatoren 110, 120 gemäß verschiedener hierin beschriebener Beispiele anzusteuern. FIG. 9 illustriert insbesondere den Verlauf der Amplitude der Signalformen 200 als Funktion der Zeit.

Das Beispiel der FIG. 9 entspricht grundsätzlich dem Beispiel der FIG. 7. Jedoch weisen in dem Beispiel der FIG. 9 die Signalbeiträge 212, 222 jeweils einen DC-Anteil 201 auf. Im Allgemeinen ist es möglich, dass lediglich einzelne der Signalbeiträge 211, 212, 221, 222 den DC-Anteil 201 aufweisen. Es wäre auch möglich, dass unterschiedliche Signalbeiträge 211, 212, 221, 222 unterschiedlich dimensionierte DC-Anteile 201 aufweisen.

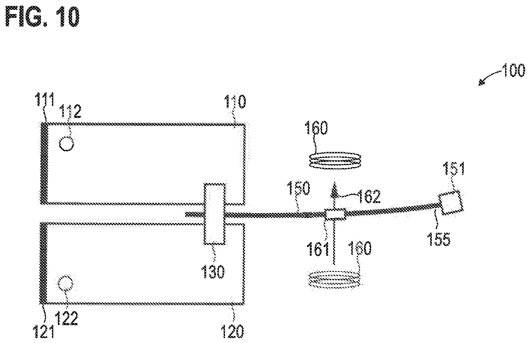

FIG. 10 illustriert Aspekte in Bezug auf eine Vorrichtung 100. Das Beispiel der FIG. 10 entspricht grundsätzlich dem Beispiel der FIG. 1. In dem Beispiel der FIG. 10 umfasst die Vorrichtung 100 weiterhin einen Magneten 161, der an der Faser 150 angebracht ist. Insbesondere ist der Magnet 161 in einem Bereich an der Faser 150 angebracht, der zwischen dem beweglichen Ende 155 und dem Verbindungsteil 130 angeordnet ist. Z.B. könnte der Magnet 161 ein ferromagnetisches Bulk-Material sein. Z.B. könnte der Magnet 161 ein Wuchtgewicht ausbilden. Z.B. könnt der Magnet 161 als Dünnfilm-Beschichtung einer Oberfläche der Faser 150 ausgebildet sein, z.B. mit einer Schichtdicke im Bereich von 20 - 500 nm. In dem Beispiel der FIG. 10 umfasst die Vorrichtung 100 auch eine Magnetfeldquelle 160. Beispielsweise könnte die Magnetfeldquelle 160 durch Spulenwicklungen implementiert sein, die zum Beispiel ein Eisenjoch aufweisen (in FIG. 10 nicht dargestellt). Mittels der Magnetfeldquelle 160 - die zum Beispiel von dem Treiber 101 angesteuert werden kann - kann es möglich sein, eine Vorspannung auf die Faser 150 aufzuprägen. Dazu kann der Treiber 101 die Magnetfeldquelle 160 mit einer Signalform ansteuern, die einen DC-Anteil aufweist und damit ein DC-Magnetfeld 162 implementiert. In dem Beispiel der FIG. 10 ist eine DC-Auslenkung der Faser 150 aufgrund des Magnetfelds 162, dass durch die Magnetfeldquelle 160 erzeugt wird, dargestellt. Diese Vorspannung der Faser 150 bewirkt eine Krümmung der Faser 150, wodurch zum Beispiel Licht mittels der Umlenkeinheit 151 in einem Ruhezustand der Faser 150 unter einem anderen Abstrahlwinkel abgestrahlt wird, als ohne Krümmung. Dadurch können zum Beispiel ein Versatz der Faser, z.B. aufgrund von Drift, und/oder Vorgaben für das Sichtfeld des entsprechenden Scanners kompensiert oder berücksichtigt werden. Diese DC-Auslenkung der Faser 150 kann dann überlagert werden von AC-Auslenkungen, die zum Beispiel durch die Signalformen 200, die von den Biegepiezoaktuatoren 110, 120 verwendet werden, aufgeprägten werden kann.

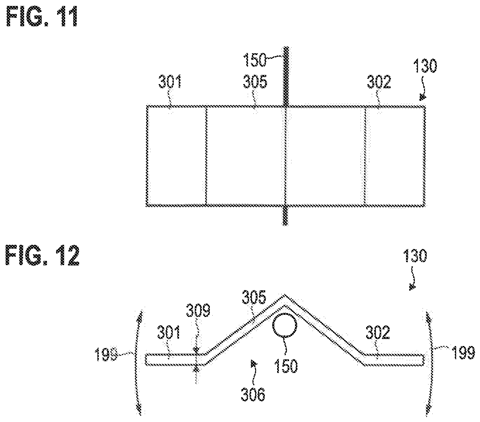

FIG. 11 illustriert Aspekte in Bezug auf das Verbindungsteil 130. FIG. 11 ist eine Aufsicht auf das Verbindungsteil 130. Das Verbindungsteil 130 weist einen Seitenbereich 301 und einen dem Seitenbereich 301 gegenüberliegenden Seitenbereich 302 auf. Dazwischen ist ein Zentralbereich 305 angeordnet. Im Zentralbereichs 305 kann eine Ausbuchtung 306 angeordnet sein, die durch eine Wölbung des Zentralbereich 305 gegenüber den Seitenbereichen 301, 302 erreicht werden kann (aus der Zeichenebene der FIG. 11 heraus oder in die Zeichenebene der FIG. 11 hinein orientiert). Die Seitenbereiche 301, 302 können Auflageflächen ausbilden, an denen die Biegepiezoaktuatoren 110, 120 angebracht sind, beispielsweise durch Kleben.

FIG. 12 illustriert Aspekte in Bezug auf das Verbindungsteil 130. FIG. 12 ist eine Aufsicht auf das Verbindungsteil 130 gemäß dem Beispiel der FIG. 11.in FIG. 12 ist ersichtlich, dass die Faser 150 im Bereich der Ausbuchtung 306 am Verbindungsteil 130 angeordnet ist. Zum Beispiel kann die Faser 150 im Bereich der Ausbuchtung 306 am Verbindungsteil 130 angeklebt sein.

Durch das Ausbilden der Ausbuchtung 306 kann eine besonders feste Kopplung zwischen dem Verbindungsteil 130 und der Faser 150 erzielt werden. Zum Beispiel kann die Ausbuchtung 300 und U-förmig ausgebildet sein. Im Bereich des "Bodens" des derart ausgebildeten U-förmigen Bereichs kann dann eine Führung für die Faser 150 entlang der Längsachse der Faser 150 bereitgestellt werden. Dies kann einen besonders großen Kraftfluss durch Auslenkung 199 der Biegepiezoaktuatoren 110, 120 gewährleisten, ohne dass Bruch auftritt.

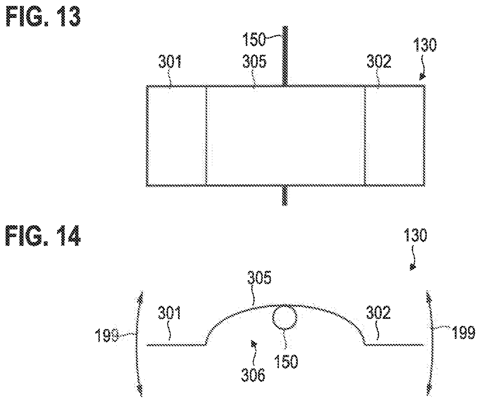

FIGs. 13 und 14 illustrieren Aspekte in Bezug auf das Verbindungsteil 130. Dabei ist FIG. 13 eine Aufsicht auf das Verbindungsteil 130 und FIG. 14 ist eine Seitenansicht des Verbindungsteil 230. Das Beispiel der FIGs. 13 und 14 entspricht grundsätzlich dem Beispiel der FIGs. 11 und 12. In dem Beispiel der FIGs. 13 und 14 ist das Verbindungsteil 130 auch U-förmig ausgebildet. Jedoch ist die Ausbuchtung 306 nicht spitz zulaufend, sondern weist eine kontinuierliche Krümmung auf. Dadurch kann die Materialstabilität des Verbindungsteil 130 gesteigert werden. Außerdem kann ein scharfer Grad vermieden werden.

Ein solcher kontinuierlicher Übergang könnte zwischen den Seitenbereichen 301, 302 und dem Zentralbereich 305 implementiert werden (in FIG. 14 nicht dargestellt).

In den verschiedenen hierin beschriebenen Beispielen wäre es möglich, dass das Verbindungsteil 130 aus Metall gefertigt ist. Beispielweise könnte das Verbindungsteil 130 aus Stahl gefertigt sein. Es wäre auch möglich, dass das Verbindungsteil 130 aus Messing gefertigt ist. Derart kann eine genügend große Stabilität für das Verbindungsteil 130 bereitgestellt werden, sodass aufgrund der Auslenkung 199 der Biegepiezoaktuatoren 110, 120 keine oder keine signifikante Materialermüdung des Verbindungsteil 130 auftritt.

In anderen Beispielen könnte das Verbindungsteil 130 aber auch aus demselben Material wie die Faser 150 gefertigt sein. Zum Beispiel könnten das Verbindungsteil 130 und die Faser 150 einstückig aus demselben Material ausgebildet werden. Dazu kann z.B. ein micromachining-Ansatz verwendet werden, bei die Faser 150 und das Verbindungsteil 130 aus einem Wafer-z.B. einem Silizium-Wafer oder einem Silizium-auf-Isolator-Wafer- freigestellt werden. Derart kann eine besonders robuste Kopplung zwischen Faser 150 und Verbindungsteil 130 bewirkt werden. Insbesondere in solchen Szenarien kann es möglich sein, dass das Verbindungsteil 130 keine Ausbuchtung aufweist, sondern Eben ausgebildet ist. Das Verbindungsteil 130 kann sich in derselben Ebene erstrecken, wie die Faser 150.

Gemäß der Erfindung ist es erstrebenswert, wenn das Verbindungsteil eine besonders geringe Dicke 309 aufweist. Erfindungsgemäß soll die Dicke 309 des Verbindungsteil 230 im Bereich von 5-150 µm liegen, optional im Bereich von 10-100 µm, weiter optional im Bereich von 40-60 µm. Derart kann erreicht werden, dass das Verbindungsteil 130 eine gewisse Elastizität aufweist und dadurch die Auslenkung 199 der Biegepiezoaktuatoren 110, 120 nicht oder nicht signifikant dämpft. Dadurch kann die Faser 150 mit einer großen Amplitude angeregt werden.

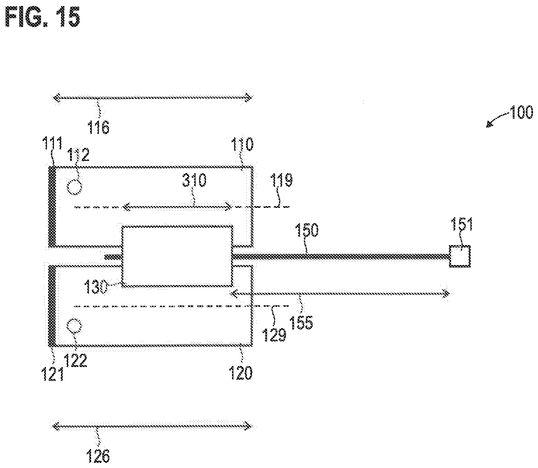

FIG. 15 illustriert Aspekte in Bezug auf die Vorrichtung 100. Insbesondere illustriert FIG. 15 Aspekte in Bezug auf eine Dimensionierung des Verbindungsteil 130 in Bezug auf die Biegepiezoaktuatoren 110, 120 bzw. die Faser 150. Das Beispiel der FIG. 15 entspricht grundsätzlich dem Beispiel der FIG. 1, jedoch ist in dem Beispiel der FIG. 15 das Verbindungsteil 130 länger dimensioniert, als in dem Beispiel der FIG. 15. Dies bedeutet, dass die Längsausdehnung 310 des Verbindungsteil 130 parallel zu den Längsachsen 119, 129 der Biegepiezoaktuatoren 110, 120 in dem Beispiel der FIG. 15 größer dimensioniert ist, als in dem Beispiel der FIG. 1.

Beispielsweise wäre es im Allgemeinen möglich, dass das Verbindungsteil 130 eine Längsausdehnung 310 entlang der Längsachsen 110, 129 der Biegepiezoaktuatoren 110, 120 aufweist, die im Bereich von 2-20 % der Längen 116, 126 der Biegepiezoaktuatoren 110, 120 liegt, optional im Bereich von 5-15 %. Durch eine solche Dimensionierung der Längsausdehnung 310 des Verbindungsteil 130 kann einerseits gewährleistet werden, dass die Auslenkung 199 der Biegepiezoaktuatoren 110, 120 durch das Verbindungsteil 130 nicht besonders stark gedämpft wird. Dadurch kann die Bewegung der Faser 150 mit einer großen Amplitude angeregt werden. Andererseits kann gewährleistet werden, dass der Kraftfluss zwischen den Biegepiezoaktuatoren 110, 120 über das Verbindungsteil 130 und auf die Faser 150 keine zu großen Verspannungen in einem kleinen Raumbereich bewirkt. Dadurch kann die Vorrichtung 100 stabiler implementiert werden.

Zusammenfassend wurden voranstehend Techniken beschrieben, welche eine besonders effiziente Anregung einer Faser ermöglichen. Die voranstehend beschriebenen Techniken ermöglichen insbesondere eine effiziente Anregung der Faser mit einer vergleichsweisen hohen Integration der entsprechenden Vorrichtung. Dies bedeutet, dass verschiedene Bewegungsfreiheitsgrade der Faser angeregt werden können, ohne dass die entsprechende Vorrichtung einen großen Platzbedarf aufweist.
In den verschiedenen hierin beschriebenen Techniken kann es insbesondere möglich sein, dass eine zur Anregung der Faser verwendete Vorrichtung eine längliche Bauform aufweist. Dies kann aufgrund der Tatsache sein, dass die Faser eine Längsachse aufweist, die sich im Wesentlichen parallel zu den Längsachsen von zwei oder mehr Biegepiezoaktuatoren erstreckt. Beispielsweise könnten in absoluten Dimensionen die Faser eine Ausdehnung im Bereich von 2-10 mm entlang ihrer Längsachse aufweisen. Entsprechend wäre es zum Beispiel möglich, dass die verwendeten Biegepiezoaktuatoren eine Ausdehnung entlang ihrer Längsachsen im Bereich von 2-20 mm aufweisen.

Basierend auf solchen Techniken kann eine besonders flexible Integration einer entsprechenden Vorrichtung in ein System erfolgen. Beispielsweise könnte eine entsprechende Vorrichtung für ein LIDAR-System in ein Kraftfahrzeug integriert werden.

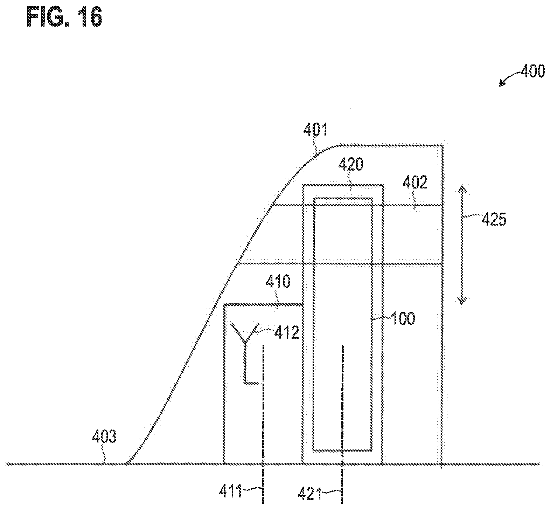

FIG. 16 illustriert Aspekte in Bezug auf ein System 400. Insbesondere illustriert FIG. 16 Aspekte in Bezug auf die Integration der Vorrichtung 100 in das System 400.

Das System 400 umfasst ein längliches Gehäuse 410 und ein weiteres längliches Gehäuse 420. Die länglichen Gehäuse 410, 420 sind parallel zueinander angeordnet. Deshalb erstreckt sich das Gehäuse 420 entlang dem Gehäuse 410. Das System 400 umfasst auch eine Hochfrequenz-Antenne 412, die in dem Gehäuse 410 angeordnet ist. Beispielweise könnte die Hochfrequenz-Antenne 412 für Radioempfang, Fernsehempfang, Satellitenempfang, und/oder Mobilfunkempfang, etc. verwendet werden.

Die Vorrichtung 100 gemäß verschiedene hierin beschriebener Beispiele ist in dem Gehäuse 420 angeordnet. Sowohl das Gehäuse 420, als auch das Gehäuse 410 sind in ein weiteres Gehäuse 401 des Systems 400 integriert. Beispielsweise könnte das Gehäuse 401 eine Haifischflossen-Form aufweisen und zum Beispiel im Bereich eines Daches eines Kraftfahrzeugs angeordnet sein. Das Gehäuse 401 und das Gehäuse 420 umfassen ein transparentes Fenster 402. Das transparente Fenster 402 kann insbesondere lichtdurchlässig für Licht sein, welches durch die Umlenkeinheit 151 der Faser 150 abgelenkt wird. Beispielweise könnte sich die Umlenkeinheit 151 im Bereich des Fensters 402 befinden.

In dem Beispiel der FIG. 16 sind auch Längsachsen 411, 421 der Gehäuse 410, 420 dargestellt. Beispielsweise wäre es möglich, dass die Längsachse 421 des Gehäuses 420 parallel zur Längsachse der Faser 150 verläuft bzw. parallel zur Längsachse 119 des Biegepiezoaktuators 110 und/oder parallel zur Längsachse 129 des Biegepiezoaktuators 120. Dadurch kann eine besonders geringe Ausdehnung des Gehäuses 420 senkrecht zur Längsachse 421 erzielt werden und damit eine hohe Integration.

Aus dem Beispiel der FIG. 16 ist ersichtlich, dass sich das Gehäuse 420 in einem exponierten Bereich 425 über das Gehäuse 410 hinaus erstreckt. Dadurch kann erreicht werden, dass das im exponierten Bereich 425 angeordnete Fenster 402 einen großen Umfang senkrecht zur Längsachse 421 aufweist, beispielsweise von nicht weniger als 50°, optional von nicht weniger als 120°, weiter optional von nicht weniger als 200°, weiter optional von 360°. Dies kann bedeuten, dass das Licht mittels der Umlenkeinheit 151 in einen großen Umfeldbereich abgestrahlt werden kann. Dies kann für LIDAR-Techniken den Vorteil großer Sichtbereiche aufweisen. Gleichzeitig wird das Umlenken des Lichts nicht durch das Gehäuse 410 blockiert.

Die Gehäuse 410, 420 weisen eine gemeinsame Grundplatte 403 auf. Die Grundplatte 403 ist gegenüberliegend zum exponierten Bereich 425 angeordnet. Beispielsweise wäre es möglich, dass die Grundplatte 403 an einer Oberfläche eines Daches eines Kraftfahrzeugs angebracht ist. Durch ein solches erhabenes Anbringen der Vorrichtung 100 kann ein besonders großer Weitblick bei mittels der Vorrichtung 100 implementierten LIDAR-Systemen erzielt werden.

Selbstverständlich können die Merkmale der vorab beschriebenen Ausführungsformen und Aspekte der Erfindung miteinander kombiniert werden. Insbesondere können die Merkmale nicht nur in den beschriebenen Kombinationen, sondern auch in anderen Kombinationen oder für sich genommen verwendet werden, ohne das Gebiet der Erfindung zu verlassen.

Zum Beispiel wurden obenstehend verschiedene Beispiele in Bezug auf Laserlicht beschrieben. Es können entsprechende Techniken aber auch für anderes Licht verwendet werden.

Ferner wurden obenstehend verschiedene Beispiele beschrieben, bei denen ein Verbindungsteil mit einer Ausbuchtung verwendet wird. In anderen Beispielen wäre es aber auch möglich, dass das Verbindungsteil eben und ohne Ausbuchtung ausgebildet ist.

## Patentansprüche

1. Vorrichtung (100) zur Anregung eines faserförmigen Elements (150), die umfasst:
- einen ersten Biegepiezoaktuator (110, 120), der plattenförmig ausgebildet ist,
- einen zweiten Biegepiezoaktuator (110, 120), der plattenförmig ausgebildet ist,
- ein Verbindungsteil (130), das zwischen dem ersten Biegepiezoaktuator (110, 120) und dem zweiten Biegepiezoaktuator (110, 120) angeordnet ist,
- ein faserförmiges Element (150), das am Verbindungsteil (130) angebracht ist, mit einem beweglichen Ende (155), welches gegenüberliegend zu dem Verbindungsteil (130) angeordnet ist, und
- einen Treiber (101), der eingerichtet ist, um den ersten Biegepiezoaktuator (110, 120) mit einer ersten Signalform (200) anzusteuern und um den zweiten Biegepiezoaktuator (110, 120) mit einer zweiten Signalform (200) anzusteuern,
wobei die erste Signalform (200) und die zweite Signalform (200) gegenphasige Signalbeiträge (211, 221) aufweisen,
**dadurch gekennzeichnet, dass** die erste Signalform (200) und die zweite Signalform (200) gleichphasige weitere Signalbeiträge (212, 222) aufweisen, und
dass das Verbindungsteil (130) plättchenförmig ausgebildet ist und eine Dicke (390) im Bereich von 5 µm bis 150 µm aufweist.

2. Vorrichtung (100) nach Anspruch 1,
wobei der erste Biegepiezoaktuator (110, 120) eine längliche Form entlang einer ersten Längsachse (119, 129) aufweist,
wobei der zweite Biegepiezoaktuator (110, 120) eine längliche Form entlang einer zweiten Längsachse (119, 129) aufweist,
wobei die erste Längsachse (119, 129) und/oder die zweite Längsachse (119, 129) einen Winkel mit einer Längsachse das faserförmigen Elements (150) einschließt, der kleiner als 20° ist, optional kleiner als 10°, weiter optional kleiner als 1°.

3. Vorrichtung (100) nach Anspruch 1 oder 2,
wobei das Verbindungsteil (130) und das faserförmige Element (150) einstückig aus demselben Material ausgebildet sind.

4. Vorrichtung (100) nach einem der voranstehenden Ansprüche, wobei die gleichphasigen weiteren Signalbeträge amplitudenmoduliert sind.

5. Vorrichtung (100) nach einem der voranstehenden Ansprüche,
wobei das Verbindungsteil (130) eine Längsausdehnung entlang einer ersten Längsachse (119, 129) des ersten Biegepiezoaktuators (110, 120) aufweist, die im Bereich von 2 - 20 % der Länge des ersten Biegepiezoaktuators (110, 120) entlang der ersten Längsachse (119, 129) ist, optional im Bereich von 5 - 15 %, und/oder
wobei das Verbindungsteil (130) eine Längsausdehnung entlang einer zweiten Längsachse (119, 129) des zweiten Biegepiezoaktuators (110, 120) aufweist, die im Bereich von 2 - 20 % der Länge des zweiten Biegepiezoaktuators (110, 120) entlang der zweiten Längsachse (119, 129) ist, optional im Bereich von 5 - 15 %.

6. Vorrichtung (100) nach einem der voranstehenden Ansprüche,
wobei der erste Biegepiezoaktuator (110, 120) eine längliche Form entlang einer ersten Längsachse (119, 129) aufweist,
wobei der zweite Biegepiezoaktuator (110, 120) eine längliche Form entlang einer zweiten Längsachse (119, 129) aufweist,
wobei sich der erste Biegepiezoaktuator (110, 120) entlang der ersten Längsachse (119, 129) und der zweite Biegepiezoaktuator (110, 120) entlang der zweiten Längsachse (119, 129) entlang einer Längsachse des faserförmigen Elements (150) hin zu einem frei beweglichen Ende des faserförmigen Elements (150) erstrecken.

7. Vorrichtung (100) nach einem der voranstehenden Ansprüche,
wobei das faserförmige Element (150) an einem ersten Ende am Verbindungsteil (130) angebracht ist,
wobei ein Spiegel (151) der Vorrichtung (100) an seiner Rückseite an einem dem ersten Ende gegenüberliegenden zweiten Ende des faserförmigen Elements (150) angebracht ist,
wobei der Treiber (101) eingerichtet ist, um eine Verdrehung des Spiegels (151) mittels der gegenphasigen Signalbeiträge (211, 221) zum Scannen von Licht mittels des optischen Elements (151) zu bewirken.

8. Vorrichtung (100) nach einem der voranstehenden Ansprüche,
wobei das faserförmige Element (150) an einem ersten Ende am Verbindungsteil (130) angebracht ist,
wobei ein optisches Element (151) an einem dem ersten Ende gegenüberliegenden zweiten Ende des faserförmigen Elements (150) angebracht ist,
wobei der Treiber (101) eingerichtet ist, um eine transversale Auslenkung des optischen Elements (151) mittels der gleichphasigen Signalbeiträge (212, 222) zu dämpfen.

9. Vorrichtung (100) nach einem der voranstehenden Ansprüche,
wobei sich der erste Biegepiezoaktuator (110, 120) und der zweite Biegepiezoaktuator (110, 120) in der Ebene erstrecken.

10. Vorrichtung (100) nach einem der voranstehenden Ansprüche, die weiterhin umfasst:
- einen Treiber (101), der eingerichtet ist, um den ersten Biegepiezoaktuator (110, 120) mit einer ersten Signalform (200) anzusteuern und um den zweiten Biegepiezoaktuator (110, 120) mit einer zweiten Signalform (200) anzusteuern,
wobei die erste Signalform (200) einen Signalbeitrag mit einer Frequenz von nicht weniger als 200 Hz aufweist, optional nicht weniger als 500 Hz,
wobei die zweite Signalform (200) einen Signalbeitrag mit einer Frequenz von nicht weniger als 200 Hz aufweist, optional nicht weniger als 500 Hz.

11. Vorrichtung (100) nach Anspruch 10,
wobei die erste Signalform (200) und/oder die zweite Signalform (200) einen DC-Anteil aufweisen.

12. Vorrichtung (100) nach einem der voranstehenden Ansprüche,
wobei das Verbindungsteil (130) zwei Seitenbereiche (302) und einen dazwischen angeordneten Zentralbereich (301) aufweist,
wobei das faserförmige Element (150) am Zentralbereich (301) angebracht ist.

13. Verfahren zum Anregen eines faserförmigen Elements (150), das sich weg von einem Verbindungsteil (130) erstreckt, welches zwischen einem plattenförmigen ersten Biegepiezoaktuator (110, 120) und einem plattenförmigen zweiten Biegepiezoaktuator (110, 120) angeordnet ist,
wobei das Verfahren umfasst:
- Ansteuern des ersten Biegepiezoaktuators (110, 120) mit einer ersten Signalform (200), und
- Ansteuern des zweiten Biegepiezoaktuators (110, 120) mit einer zweiten Signalform (200),
**dadurch gekennzeichnet, dass** die erste Signalform (200) und die zweite Signalform (200) gleichphasige Signalbeiträge (212, 222) und gegenphasige Signalbeiträge (211, 221) aufweisen, und
dass das Verbindungsteil (130) plättchenförmig ausgebildet ist und eine Dicke (390) im Bereich von 5 µm bis 150 µm aufweist.

14. Verfahren nach Anspruch 13, das weiterhin umfasst:
wobei das faserförmige Element (150) an einem ersten Ende am Verbindungsteil (130) angebracht ist,
wobei ein optisches Element (151) an einem dem ersten Ende gegenüberliegenden zweiten Ende des faserförmigen Elements (150) angebracht ist,
wobei das Verfahren weiterhin umfasst:
- Bewirken einer Verdrehung des optischen Elements (151) mittels der gegenphasigen Signalbeiträge (211, 221) zum Scannen von Licht mittels des optischen Elements (151).

15. Verfahren nach Anspruch 13 oder 14,
wobei das faserförmige Element (150) an einem ersten Ende am Verbindungsteil (130) angebracht ist,
wobei ein optisches Element (151) an einem dem ersten Ende gegenüberliegenden zweiten Ende des faserförmigen Elements (150) angebracht ist,
wobei das Verfahren weiterhin umfasst:
- Dämpfen einer transversalen Auslenkung des optischen Elements (151) mittels der gleichphasigen Signalbeiträge (212, 222).

## Claims

1. A device (100) for exciting a fiber-shaped element (150), which comprises:
- a first piezo bender actuator (110, 120) which is designed in the shape of a plate,
- a second piezo bender actuator (110, 120) which is designed in the shape of a plate,
- a connecting part (130) which is arranged between the first piezo bender actuator (110, 120) and the second piezo bender actuator (110, 120),
- a fiber-shaped element (150) which is attached to the connecting part (130), with a movable end (155) which is arranged opposite the connecting part (130), and
- a driver (101) which is configured to actuate the first piezo bender actuator (110, 120) with a first signal shape (200) and to actuate the second piezo bender actuator (110, 120) with a second signal shape (200),
wherein the first signal shape (200) and the second signal shape (200) comprise out-of-phase signal contributions (211, 221),
**characterized in that** the first signal shape (200) and the second signal shape (200) comprise in-phase additional signal contributions (212, 222), and
**in that** the connecting part (130) is designed in the shape of a small plate and has a thickness (390) in the range of 5 µm to 150 µm.

2. The device (100) according to Claim 1,
wherein the first piezo bender actuator (110, 120) has an elongate shape along a first longitudinal axis (119, 129),
wherein the second piezo bender actuator (110, 120) has an elongate shape along a second longitudinal axis (119, 129),
wherein the first longitudinal axis (119, 129) and/or the second longitudinal axis (119, 129) enclose/encloses an angle with a longitudinal axis of the fiber-shaped element (150), which is smaller than 20°, optionally smaller than 10°, moreover optionally smaller than 1°.

3. The device (100) according to Claim 1 or 2,
wherein the connecting part (130) and the fiber-shaped element (150) are designed to form a single piece made of the same material.

4. The device (100) according to any one of the preceding claims,
wherein the in-phase additional signal contributions are amplitude modulated.

5. The device (100) according to any one of the preceding claims,
wherein the connecting part (130) has a longitudinal extension along a first longitudinal axis (119, 129) of the first piezo bender actuator (110, 120), which is in the range of 2-20% of the length of the first piezo bender actuator (110, 120) along the first longitudinal axis (119, 129), optionally in the range of 5-15%, and/or
wherein the connecting part (130) has a longitudinal extension along a second longitudinal axis (119, 129) of the second piezo bender actuator (110, 120), which is in the range of 2-20% of the length of the second piezo bender actuator (110, 120) along the second longitudinal axis (119, 129), optionally in the range of 5-15%.

6. The device (100) according to any one of the preceding claims,
wherein the first piezo bender actuator (110, 120) has an elongate shape along a first longitudinal axis (119, 129),
wherein the second piezo bender actuator (110, 120) has an elongate shape along a second longitudinal axis (119, 129),
wherein the first piezo bender actuator (110, 120) along the first longitudinal axis (119, 129) and the second piezo bender actuator (110, 120) along the second longitudinal axis (119, 129) extend along a longitudinal axis of the fiber-shaped element (150) toward a freely movable end of the fiber-shaped element (150).

7. The device (100) according to any one of the preceding claims,
wherein the fiber-shaped element (150) is attached on a first end to the connecting part (130),
wherein a mirror (151) of the device (100) is attached on its rear side to a second end of the fiber-shaped element (150), which is opposite the first end,
wherein the driver (101) is configured to bring about a twisting of the mirror (151) by means of the out-of-phase signal contributions (211, 221) for scanning light by means of the optical element (151).

8. The device (100) according to any one of the preceding claims,
wherein the fiber-shaped element (150) is attached on a first end to the connecting part (130),
wherein an optical element (151) is attached on a second end of the fiber-shaped element (150), which is opposite the first end,
wherein the driver (101) is configured to damp a transverse deflection of the optical element (151) by means of the in-phase signal contributions (212, 222).

9. The device (100) according to any one of the preceding claims,
wherein the first piezo bender actuator (110, 120) and the second piezo bender actuator (110, 120) extend in the plane.

10. The device (100) according to any one of the preceding claims, which moreover comprises:
- a driver (101) which is configured to actuate the first piezo bender actuator (110, 120) with a first signal shape (200) and to actuate the second piezo bender actuator (110, 120) with a second signal shape (200),
wherein the first signal shape (200) has a signal contribution with a frequency of no less than 200 Hz, optionally no less than 500 Hz,
wherein the second signal shape (200) has a signal contribution with a frequency of no less than 200 Hz, optionally no less than 500 Hz.

11. The device (100) according to Claim 10,
wherein the first signal shape (200) and/or the second signal shape (200) has/have a DC component.

12. The device (100) according to any one of the preceding claims,
wherein the connecting part (130) comprises two side areas (302) and a central area (301) arranged in between,
wherein the fiber-shaped element (150) is attached to the central area (301).

13. A method for exciting a fiber-shaped element (150) which extends away from a connecting part (130) which is arranged between a plate-shaped first piezo bender actuator (110, 120) and a plate-shaped second piezo bender actuator (110, 120),
wherein the method comprises:
- actuating the first piezo bender actuator (110, 120) with a first signal shape (200), and
- actuating the second piezo bender actuator (110, 120) with a second signal shape (200),
**characterized in that** the first signal shape (200) and the second signal shape (200) have in-phase signal contributions (212, 222) and out-of-phase signal contributions (211, 221), and
**in that** the connecting part (130) is designed in the shape of a small plate and has a thickness (390) in the range of 5 µm to 150 µm.

14. The method according to Claim 13, which moreover comprises:
wherein the fiber-shaped element (150) is attached on a first end to the connecting part (130),
wherein an optical element (151) is attached on a second end of the fiber-shaped element (150), which is opposite the first end,
wherein the method moreover comprises:
- bringing about a twisting of the optical element (151) by means of the out-of-phase signal contributions (211, 221) for scanning light by means of the optical element (151).

15. The method according to Claim 13 or 14,
wherein the fiber-shaped element (150) is attached on a first end to the connecting part (130),
wherein an optical element (151) is attached on a second end of the fiber-shaped element (150), which is opposite the first end,
wherein the method moreover comprises:
- damping a transverse deflection of the optical element (151) by means of the in-phase signal contributions (212, 222).

## Revendications

1. Dispositif (100) pour la stimulation d'un élément fibreux (150) qui comprend :
- un premier actionneur piézoélectrique à flexion (110, 120) qui est réalisé en forme de plaque,
- un second actionneur piézoélectrique à flexion (110, 120) qui est réalisé en forme de plaque,
- une pièce de liaison (130) qui est disposée entre le premier actionneur piézoélectrique à flexion (110, 120) et le second actionneur piézoélectrique à flexion (110, 120),
- un élément fibreux (150) qui est installé sur la pièce de liaison (130), avec une extrémité mobile (155) qui est disposée en face de la pièce de liaison (130), et
- un élément d'entraînement (101) qui est adapté pour commander le premier actionneur piézoélectrique à flexion (110, 120) avec une première forme de signal (200) et pour commander le second actionneur piézoélectrique à flexion (110, 120) avec une seconde forme de signal(200),
dans lequel la première forme de signal (200) et la seconde forme de signal (200) présentent des composantes de signal en opposition de phases (211, 221),
**caractérisé en ce que** la première forme de signal (200) et la seconde forme de signal (200) présentent d'autres composantes de signal en phase (212, 222), et
**en ce que** la pièce de liaison (130) est réalisée en forme de plaquette et présente une épaisseur (390) dans la plage de 5 µm à 150 µm.

2. Dispositif (100) selon la revendication 1,
dans lequel le premier actionneur piézoélectrique à flexion (110, 120) présente une forme allongée le long d'un premier axe longitudinal (119, 129),
dans lequel le second actionneur piézoélectrique à flexion (110, 120) présente une forme allongée le long d'un second axe longitudinal (119, 129),
dans lequel le premier axe longitudinal (119, 129) et/ou le second axe longitudinal (119, 129) forment un angle avec un axe longitudinal de l'élément fibreux (150) qui est inférieur à 20 °, facultativement inférieur à 10 °, en outre facultativement inférieur à 1 °.

3. Dispositif (100) selon la revendication 1 ou 2,
dans lequel la pièce de liaison (130) et l'élément fibreux (150) sont réalisés d'un seul tenant à partir du même matériau.

4. Dispositif (100) selon l'une des revendications précédentes,
dans lequel les autres composantes de signal en phase sont modulées en amplitude.

5. Dispositif (100) selon l'une des revendications précédentes,
dans lequel la pièce de liaison (130) présente un allongement longitudinal le long d'un premier axe longitudinal (119, 129) du premier actionneur piézoélectrique à flexion (110, 120) qui est dans la plage de 2 à 20 % de la longueur du premier actionneur piézoélectrique à flexion (110, 120) le long du premier axe longitudinal (119, 129), facultativement dans la plage de 5 à 15 %, et/ou
dans lequel la pièce de liaison (130) présente un allongement longitudinal le long d'un second axe longitudinal (119, 129) du second actionneur piézoélectrique à flexion (110, 120) qui est dans la plage de 2 à 20 % de la longueur du second actionneur piézoélectrique à flexion (110, 120) le long du second axe longitudinal (119, 129), facultativement dans la plage de 5 à 15 %.

6. Dispositif (100) selon l'une des revendications précédentes,
dans lequel le premier actionneur piézoélectrique à flexion (110, 120) présente une forme allongée le long d'un premier axe longitudinal (119, 129),
dans lequel le second actionneur piézoélectrique à flexion (110, 120) présente une forme allongée le long d'un second axe longitudinal (119, 129),
dans lequel le premier actionneur piézoélectrique à flexion (110, 120) le long du premier axe longitudinal (119, 129) et le second actionneur piézoélectrique à flexion (110, 120) le long du second axe longitudinal (119, 129) s'étendent le long d'un axe longitudinal de l'élément fibreux (150) en direction d'une extrémité librement mobile de l'élément fibreux (150).

7. Dispositif (100) selon l'une des revendications précédentes,
dans lequel l'élément fibreux (150) est installé au niveau d'une première extrémité sur la pièce de liaison (130),
dans lequel un miroir (151) du dispositif (100) est installé sur sa face arrière au niveau d'une seconde extrémité de l'élément fibreux (150) en face de la première extrémité,
dans lequel l'élément d'entraînement (101) est adapté pour appliquer une torsion du miroir (151) au moyen des composantes de signal en opposition de phases (211, 221) pour un balayage de lumière au moyen de l'élément optique (151).

8. Dispositif (100) selon l'une des revendications précédentes,
dans lequel l'élément fibreux (150) est installé au niveau d'une première extrémité sur la pièce de liaison (130),
dans lequel un élément optique (151) est disposé au niveau d'une seconde extrémité l'élément fibreux (150) en face de à la première extrémité,
dans lequel l'élément d'entraînement (101) est adapté pour amortir pour amortir une déformation transversale de l'élément optique (151) au moyen des composantes de signal en phase (212, 222) .

9. Dispositif (100) selon l'une des revendications précédentes,
dans lequel le premier élément piézoélectrique à flexion (110, 120) et le second élément piézoélectrique à flexion (110, 120) s'étendent dans le plan.

10. Dispositif (100) selon l'une des revendications précédentes, qui comprend en outre :
- un élément d'entraînement (101) qui est adapté pour commander le premier actionneur piézoélectrique à flexion (110, 120) avec une première forme de signal (200) et pour commander le second actionneur piézoélectrique à flexion (110, 120) avec une seconde forme de signal (200),
dans lequel la première forme de signal (200) présente une composante de signal avec une fréquence de pas moins de 200 Hz, facultativement de pas moins de 500 Hz,
dans lequel la seconde forme de signal (200) présente une composante de signal avec une fréquence de pas moins de 200 Hz, facultativement de pas moins de 500 Hz.

11. Dispositif (100) selon la revendication 10,
dans lequel la première forme de signal (200) et/ou la seconde forme de signal (200) présentent une part à courant continu.

12. Dispositif (100) selon l'une des revendications précédentes,
dans lequel la pièce de liaison (130) présente deux zones latérales (302) et une zone centrale (301) disposée entre celles-ci,
dans lequel l'élément fibreux (150) est installé au niveau de la zone centrale (301).

13. Procédé pour la stimulation d'un élément fibreux (150) qui s'étend en s'éloignant d'une pièce de liaison (130), lequel est disposé entre un premier élément piézoélectrique à flexion en forme de plaque (110, 120) et un second élément piézoélectrique à flexion en forme de plaque (110, 120),
dans lequel le procédé comprend :
- la commande du premier actionneur piézoélectrique à flexion (110, 120) avec une première forme de signal (200),et
- la commande du second actionneur piézoélectrique (110, 120) avec une seconde forme de signal (200),
**caractérisé en ce que** la première forme de signal (200) et la seconde forme de signal (200) présentent des composantes de signal en phase (212, 222) et des composantes de signal en opposition de phases (211, 221), et
**en ce que** la pièce de liaison (130) est réalisée en forme de plaquette et présente une épaisseur (390) dans la plage de 5 µm à 150 µm.

14. Procédé selon la revendication 13, qui comprend en outre :
dans lequel l'élément fibreux (150) est installé au niveau d'une première extrémité sur la pièce de liaison (130),
dans lequel un élément optique (151) est installé au niveau d'une seconde extrémité de l'élément fibreux (150) en face de la première extrémité,
dans lequel le procédé comprend en outre :
- l'application d'une torsion de l'élément optique (151) au moyen des composantes de signal en opposition de phases (211, 221) pour un balayage de lumière au moyen de l'élément optique (151).

15. Procédé selon la revendication 13 ou 14,
dans lequel l'élément fibreux (150) est installé au niveau d'une première extrémité sur la pièce de liaison (130),
dans lequel un élément optique (151) est installé au niveau d'une seconde extrémité de l'élément fibreux (150) en face de la première extrémité,
dans lequel le procédé comprend en outre :
- l'amortissement d'une déformation transversale de l'élément optique (151) au moyen des composantes de signal en phase (212, 222) .
